# EUROPÄISCHE PATENTSCHRIFT

(11) **EP 2 935 210 B1**
(45) Veröffentlichungstag und Bekanntmachung des Hinweises auf die Patenterteilung: **16.11.2016**
(21) Anmeldenummer: 13814082.7
(22) Anmeldetag: 17.12.2013
(51) Int. Cl.: C07D 201/16

(54) **VERFAHREN ZUR HERSTELLUNG VON GEREINIGTEM CAPROLACTAM AUS DER BECKMANN-UMLAGERUNG VON CYCLOHEXANONOXIM**
PROCESS FOR THE PREPARATION OF PURIFIED CAPROLACTAM FROM THE BECKMAN REARRANGEMENT OF CYCLOHEXANOXIM
PROCÉDÉ DE FABRICATION DE CAPROLACTAM PURIFIÉ À PARTIR DU RÉARRANGEMENT DE BECKMANN DE CYCLOHEXANE-OXIME

(30) Priorität: 19.12.2012 EP 12198086
(43) Veröffentlichungstag der Anmeldung: 28.10.2015
(73) Patentinhaber: BASF SE, 67056 Ludwigshafen am Rhein (DE)
(72) Erfinder: FISCHER, Rolf Hartmut, 69121 Heidelberg (DE); GEHRKEN, Henning-Peter, 68549 Ilvesheim (DE); ANSMANN, Andreas, 69168 Wiesloch (DE); MÜLLER, Matthias, 64319 Pfungstadt (DE); MILLER, Christian, 67146 Deidesheim (DE)
(86) Internationale Anmeldenummer: PCT/EP2013/076829
(87) Internationale Veröffentlichungsnummer: WO 2014/095807

(56) Entgegenhaltungen:
- EP-A1- 0 022 161
- WO-A1-03/018550

## Beschreibung

Die vorliegende Anmeldung betrifft ein Verfahren zur Reinigung von Caprolactam bzw. Herstellung von gereinigtem Caprolactam, das durch Beckmann-Umlagerung von Cyclohexanonoxim mit konzentrierter Schwefelsäure oder Oleum gewonnen wird. Das für die Oximierung von Cyclohexanon benötigte Hydroxylammoniumsulfat wird dabei vorzugsweise durch Hydrierung von Stickstoffmonoxid mit Wasserstoff in verdünnter Schwefelsäure an Palladium oder Platin enthaltenden Katalysatoren hergestellt. Die Oximierung von Cyclohexanon erfolgt mit Hydroxylammoniumsulfat unter ständiger Zugabe von Ammoniak.

Zur Herstellung von Caprolactam durch Umlagerung von Cyclohexanonoxim mit Schwefelsäure oder Oleum in der Flüssigphase zu Caprolactamsulfat werden großtechnisch zwei Verfahren durchgeführt. Sie unterscheiden sich vor allem in der Herstellung des HydroxylammoniumSalzes und des Cyclohexanonoxims durch die Bedingungen in der Oximierung und dementsprechend auch in der Reinigung des bei der Beckmann-Umlagerung erhaltenen Caprolactamsulfats zu Reincaprolactam (J. Ritz et al., Ullmann's Encyclopedia of Industrial Chemistry, 6. Aufl., Band 6, Seiten 185 bis 205).

Es ist bekannt, Nitrationen nach dem so genannten HPO-Verfahren in Gegenwart eines Palladium-Katalysators und einer Phosphatpuffer-Lösung mit Wasserstoff zu einem Hydroxylammonium-Salz zu hydrieren, das dann mit Cyclohexanon zu Cyclohexanonoxim umgesetzt wird. Die freiwerdende Phosphatpuffer-Lösung wird erneut mit Salpetersäure versetzt und in die Hydrierung zurückgeführt (Hans-Jürgen Arpe, Industrielle Organische Chemie, Wiley-VCH-Verlag, 6. Aufl., Seiten 280 bis 283).

Die Beckmann-Umlagerung des Cyclohexanonoxims wird mit Schwefelsäure oder Oleum durchgeführt. Das dabei entstehende Caprolactamsulfat und überschüssige Schwefelsäure werden mit Ammoniak zu Caprolactam und Ammoniumsulfat umgesetzt.

Aus EP 1 423 361 B1 (DSM), Seite 5, [0031] geht hervor, dass nach Oximierung und Beckmann-Umlagerung das bei der Umsetzung des Caprolactamsulfats mit Ammoniak erhaltene Caprolactam durch Extraktionen abgetrennt wird. Der Extraktstrom wird in einer Reihe von Verfahrensschritten gereinigt. Diese Schritte schließen die Reinigung durch Ionentausch und durch katalytische Hydrierung ein. Eine Hydrierung erfolgt, um ungesättigte 7-Ringlactame in Caprolactam umzuwandeln und so die Caprolactam-Qualität zu verbessern, wie z. B. in WO 03/045911 beschrieben.

Der etwa 85 Gew.-% Caprolactam, etwa 15 Gew.-% Wasser und kleine Mengen an Verunreinigungen enthaltende Produktstrom wird in einer Serie von Verdampfern entwässert, wobei die Temperaturen in den Verdampfern zwischen 80 und 125°C liegen. Die gesamte Verweilzeit in den Verdampfern beträgt 3 Stunden.

Die Wasserabtrennung erfolgt in Gegenwart von insgesamt weniger als 20 mmol Natronlauge pro kg Caprolactam. Hierzu wird die Natronlauge dem Caprolactam vor den Verdampfern hinzugefügt. Sie soll sich mit Caprolactam zum Na-Salz der 6-Aminocapronsäure umsetzen. Dieses Salz besitzt nach DD-A-202870 den gleichen Reinigungseffekt wie Natronlauge, bewirkt aber im Gegensatz zu Natronlauge keine Caprolactam-Oligomerisierung.

Der nur noch 0,5 Gew.-% Wasser enthaltende Caprolactam-Strom wird in zwei Schritten bei vermindertem Druck destilliert. Im ersten Schritt werden niedrigsiedende Verunreinigungen und Wasser in einer ersten Destillationskolonne bei einer Sumpftemperatur von 175°C, einem Druck von 5,2 kPa und einer Verweilzeit von mehreren Minuten abgetrennt. In einer zweiten Destillationskolonne werden hochsiedende Verunreinigungen in einer Kolonne bei einer Sumpftemperatur von 133°C, einem Druck von 1,2 kPa und einer Verweilzeit von einer Stunde abgetrennt.

Nachteilig an der Aufarbeitung des Reaktionsaustrags der Beckmann-Umlagerung gemäß EP 1 423 361 B1 ist die große Zahl von Reinigungsschritten: Caprolactam-Extraktion, Caprolactam-Rückextraktion, Ionentausch, katalytische Hydrierung, Entwässerung, destillative CaprolactamReinigung. Hierdurch entstehen hohe Investitions-, Betriebs- und Reparaturkosten. Besonders hervorzuheben sind dabei die Hydrierung, deren Katalysator eine hohe Standzeit besitzen muss und die Ionentauscher, die regelmäßig regeneriert werden müssen. Zudem wird kein Caprolactam erhalten, das der Standardspezifikation entspricht, da der E₂₉₀-Wert über 0,05 liegt.

EP-A-0 022 161 beschreibt ein Verfahren zur Herstellung und Reinigung von Caprolactam, bei dem nach der Extraktion und Abtrennung des Extraktionsmittels und vor der Destillation 14,9 mmol/kg Caprolactam an Natronlauge zugesetzt werden. Eine Zugabe von 12 kg/h 25 gew.%iger Natronlauge entspricht 3000 g/h Natronlauge. Bei einem Molekulargewicht von 40 g/mol entspricht dies 75 mol/h. 5300 kg/h 95 gew.-%iges Extrakt-Caprolactam entspricht 5035 kg/h Caprolactam. Es werden demnach 75 mol/5035 kg Caprolactam an Natronlauge zugesetzt, im Ergebnis 14,9 mmol/kg.

Es bestand daher die Aufgabe, ein Verfahren zur Aufarbeitung und Reinigung von Caprolactam, hergestellt durch Beckmann-Umlagerung von Cyclohexanonoxim mit Schwefelsäure oder Oleum, bereitzustellen, das mit einer geringeren Zahl von Reinigungsschritten auskommt, das geringere Betriebskosten verursacht und vorzugsweise Caprolactam einer höheren Qualität liefert, das der Standardspezifikation entspricht.

Die Aufgabe wird erfindungsgemäß gelöst durch ein Verfahren zur Herstellung von gereinigtem Caprolactam, umfassend die Schritte
a) Extraktion von Roh-Caprolactam, erhalten aus der Beckmann-Umlagerung von Cyclohexanonoxim, mit einem organischen Extraktionsmittel,
b) Abtrennung der organischen Phase aus Schritt a),
c) destillative Abtrennung des organischen Extraktionsmittels von der organischen Phase aus Schritt b) unter Erhalt von wasserhaltigem Extraktlactam, wobei vor der destillativen Abtrennung wässrige Alkalihydroxid-Lösung in einer Menge von 0,5 bis 6 mmol Alkalihydroxid/kg Caprolactam zugesetzt wird,
d) Zusetzen von 0 bis 30 mmol Alkalihydroxid/kg Caprolactam zum wasserhaltigen Extraktlactam aus Schritt c),
e) destillatives Entwässern des mit Alkalihydroxid, vorzugsweise NaOH, versetzten wasserhaltigen Extraktlactams aus Schritt d),
f) Befreien des Caprolactams aus Schritt e) von niedriger und höher als Caprolactam siedenden Nebenprodukten durch Destillation,
wobei in Schritten c) und d) zusammen mindestens 1,5 mmol Alkalihydroxid/kg Caprolactam zugesetzt werden. Die insgesamt im Verfahren zugesetzte Alkalihydroxid-Menge beträgt demnach mindestens 2 mmol/kg Caprolactam.

Die Mindestmenge an Alkalihydroxid in Schritten c) und d) zusammen liegt vorzugsweise im Bereich von 1,5 bis 15 mmol Alkalihydroxid/kg Caprolactam.

Als Alkalihydroxid werden bevorzugt NaOH oder KOH, insbesondere NaOH, eingesetzt.

Dabei werden vorzugsweise in Schritt d) 1,5 bis 30 mmol, besonders bevorzugt 1,75 bis 20 mmol, insbesondere 1,75 bis 14 mmol, speziell 1,75 bis 10 mmol Alkalihydroxid, vorzugsweise NaOH/kg Caprolactam zugesetzt.

Vorzugsweise beträgt in Schritt e) die Verweilzeit weniger als 30 Minuten, besonders bevorzugt weniger als 20 Minuten.

Vorzugsweise ist das organische Lösungsmittel in Schritt a) Toluol oder Benzol.

Vorzugsweise überschreitet in Schritt e) und/oder f) die Sumpftemperatur 160°C, besonders bevorzugt 150°C nicht.

Vorzugsweise enthält das entwässerte Caprolactam am Ende von Schritt e) 0,15 bis 0,25 Gew.- % Wasser.

Vorzugsweise beträgt dabei der Druck in Schritt e) 20 bis 100 mbar, besonders bevorzugt 30 bis 60 mbar (Kopfdruck) in mindestens einer Destillationsstufe.

Vorzugsweise beträgt der Druck in Schritt f) 2 bis 20 mbar, besonders bevorzugt 4 bis 10 mbar, insbesondere 5 bis 8 mbar (Kopfdruck).

Vorzugsweise werden in Schritt c) 1 bis 4 mmol, besonders bevorzugt 2 bis 3 mmol, insbesondere 2,5 mmol Alkalihydroxid/kg Caprolactam zugesetzt.

Gemäß einer Ausführungsform der Erfindung beträgt die Gesamtmenge des in Schritten c) und d) zugegebenen Alkalihydroxids maximal 14 mmol, bevorzugt maximal 12,5 mmol Alkalihydroxid, vorzugsweise NaOH/kg Caprolactam.

Das in der Beckmann-Umlagerung eingesetzte Cyclohexanonoxim kann durch Oximierung von Cyclohexanon mit Hydroxylammoniumsulfat hergestellt werden. Das Hydroxylammoniumsulfat wird dabei vorzugsweise durch katalytische Hydrierung von Stickstoffmonoxid in Gegenwart von Schwefelsäure erhalten.

Die einzelnen Schritte zur Herstellung des Caprolactams und zur Aufreinigung werden nachfolgend näher erläutert.

### Beckmann-Umlagerung von Cyclohexanonoxim (a)

Das für die Herstellung von Cyclohexanonoxim benötigte Cyclohexanon kann durch Cyclohexan-Oxidation mit Luft zu einem Gemisch aus Cyclohexanol und Cyclohexanon ("Anolon"), destillative Trennung des Anolons in Cyclohexanol und Cyclohexanon und Dehydrierung des Cyclohexanols zu Cyclohexanon gewonnen werden.

Es ist weiterhin möglich, Phenol einstufig zu Cyclohexanon zu hydrieren.

Schließlich kann Cyclohexanon durch Hydratisierung von Cyclohexen zu Cyclohexanol und anschließende Dehydrierung des Cyclohexanols hergestellt werden.

Das für die Oximierung von Cyclohexanon eingesetzte Hydroxylammoniumsulfat wird vorzugsweise durch Hydrierung von Stickstoffmonoxid mit Wasserstoff in Gegenwart von (verdünnter) Schwefelsäure an Platin oder Palladium enthaltenden Katalysatoren gewonnen.

Die, vorzugsweise bei der Hydrierung von Stickstoffmonoxid erhaltene Hydroxylammoniumsulfat-Lösung wird mit Cyclohexanon und Ammoniak, d. h. unter pH-kontrollierten Bedingungen, zu Cyclohexanonoxim und Ammoniumsulfat umgesetzt.

Zur Beckmann-Umlagerung des Cyclohexanonoxims wird kontinuierlich mit konzentrierter (98%-iger) Schwefelsäure oder Oleum (überschüssiges Schwefeltrioxid enthaltende Schwefelsäure) umgesetzt.

Dann werden Oxim-Schmelze und Oleum unter Kühlung in schon umgelagertes Produkt eingeleitet. Das bei der Beckmann-Umlagerung in schneller Reaktion entstehende Caprolactamsulfat und überschüssige Schwefelsäure werden mit wässrigem Ammoniak neutralisiert. Dabei entsteht ein zweiphasiges Gemisch, das als Unterphase wässriges Ammoniumsulfat und als Oberphase 60 bis 80 Gew.-% Caprolactam und 20 bis 40 Gew.-% Wasser enthält.

Die beiden flüssigen Phasen werden getrennt. Die Ammoniumsulfat-Phase wird entwässert und kristallisiert.

### Caprolactam-Extraktion und Extraktionsmittel-Rückführung (a), (b), (c)

Die wässrige Roh-Caprolactam-Phase wird mit einem organischen Lösungsmittel, bevorzugt Toluol oder Benzol, extrahiert. Dabei werden zweiphasige Extrakte erhalten. Als Oberphase fallen Lösungen von Caprolactam in Toluol oder Benzol an. Als Unterphase fällt von Caprolactam befreites Wasser an, das an anderer Stelle des Verfahrens eingesetzt werden kann oder entsorgt wird.

Vor der destillativen Trennung von Extraktionsmittel und Caprolactam wird dem Extrakt vorzugweise wässrige Alkalihydroxid-Lösung zugesetzt, die verdünnt sein kann. Als Alkalihydroxide kommen dabei vorzugsweise Natriumhydroxid und Kaliumhydroxid, insbesondere Natriumhydroxid in Frage. Unter einer Natriumhydroxid-Lösung ist eine 1- bis 30 gew.-%-ige, bevorzugt 2 bis 10 gew.-%-ige, insbesondere 2,5- bis 3 gew.-%-ige wässrige Lösung zu verstehen. Typischerweise wird das Alkalihydroxid in einer Menge von 0,5 bis 6 mmol/kg Caprolactam, vorzugsweise 1 bis 4 mmol/kg Caprolactam, eingesetzt.

Das Extraktionsmittel wird abdestilliert und in die Extraktionsstufe zurückgeführt. Die destillative Abtrennung des Extraktionsmittels kann in jeder geeigneten, dem Fachmann bekannten Vorrichtung durchgeführt werden. Für diese Destillation geeignet sind Apparaturen, wie sie beispielsweise in: Kirk-Othmer, Encyclopedia of Chemical Technology, 4. Aufl., Vol. 8, John Wiley & Sons, New York, 1996, Seiten 334 bis 348, beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdampfer, Naturumlaufverdampfer oder Zwangsumlaufentspannungsverdampfer. Die Destillation kann in mehreren, wie zwei oder drei Apparaturen, vorzugsweise in einer Apparatur durchgeführt werden.

Für geeignete Bedingungen kann zudem auf EP-B-1 423 361 verwiesen werden. Eine Reinigung des Roh-Caprolactams oder Extraktlactams oder Caprolactams durch Reextraktion und Behandeln mit einem Ionentauscher und/oder durch Hydrierung erfolgt erfindungsgemäß nicht.

### Caprolactam-Entwässerung (d), (e)

Das nach destillativer Abtrennung des Extraktionsmittels erhaltene Extraktlactam enthält 70 bis 99 Gew.-%, bevorzugt 85 bis 99, insbesondere 90 bis 98 Gew.-%, Caprolactam und 1 bis 30 Gew.-%, bevorzugt 1 bis 15 Gew.-%, insbesondere 2 bis 10 Gew.-% Wasser.

Zu diesem wasserhaltigen Extraktlactam werden 0 bis 30, vorzugsweise 1,5 bis 30, weiter bevorzugt 1,75 bis 20, bevorzugt 1,75 bis 10, mehr bevorzugt 1,75 bis 7,5, besonders bevorzugt 4 bis 6 mmol Alkalihydroxid, vorzugsweise Natronlauge pro Kilogramm Caprolactam im Extraktlactam, vorzugsweise in Form von 1 bis 30 Gew.-%-iger, bevorzugt 2 bis 10, Gew.-%-iger wässriger Natronlauge zudosiert. Das Caprolactam wird dann destillativ entwässert, wobei Sumpftemperaturen von 160°C vorzugsweise nicht überschritten werden. Gearbeitet wird vorzugsweise im Druckbereich von 20 bis 100 mbar in zumindest einer Destillationsstufe. Die GesamtVerweilzeit beträgt vorzugsweise weniger als 30 Minuten, besonders bevorzugt weniger als 20 Minuten.

Das weitgehend entwässerte Caprolactam enthält vorzugsweise noch 0,15 bis 0,25 Gew.-% Wasser.

### Caprolactam-Destillation (f)

Das weitgehend entwässerte Caprolactam wird anschließend so bei vermindertem Druck destilliert, dass die Sumpftemperatur 160°C nicht überschreitet. Der Druck beträgt dabei vorzugsweise 2 bis 20 mbar, besonders bevorzugt 4 bis 10 mbar, insbesondere 5 bis 8 mbar. Bei der Destillation wird das Caprolactam von niedriger und von höher als Caprolactam siedenden Nebenprodukten befreit.

Die Caprolactam-Destillation kann in jeder geeigneten, dem Fachmann bekannten Vorrichtung durchgeführt werden. Für diese Destillation geeignet sind Apparaturen, wie sie beispielsweise in: Kirk-Othmer, Encyclopedia of Chemical Technology, 4. Aufl., Vol. 8, John Wiley & Sons, New York, 1996, Seiten 334 bis 348, beschrieben sind, wie Siebbodenkolonnen, Glockenbodenkolonnen, Packungskolonnen, Füllkörperkolonnen oder einstufige Verdampfer, wie Fallfilmverdampfer, Dünnschichtverdampfer, Flashverdampfer, Mehrphasenwendelrohrverdapfer, Naturumlaufverdampfer oder Zwangsumlaufentspannungsverdampfer.

Die Erfindung wird durch die nachstehenden Beispiele näher erläutert.

### Beispiel 1

Zur Herstellung von weitgehend wasserfreiem Caprolactam in einem kontinuierlichen Verfahren zur Herstellung von Caprolactam durch Beckmann-Umlagerung von Cyclohexanonoxim wurde zunächst Cyclohexanon mit Hydroxylammoniumsulfat-Lösung, hergestellt durch Hydrierung von Stickstoffmonoxid mit Wasserstoff in Gegenwart von verdünnter Schwefelsäure und Palladium enthaltenden Katalysatoren, zu Cyclohexanonoxim umgesetzt. Überschüssige Schwefelsäure wurde mit ständig zugegebenem Ammoniak neutralisiert, und das entstandene Ammoniumsulfat wurde abgetrennt.

Das so erhaltene Cyclohexanonoxim wurde mit Oleum zu Caprolactamsulfat umgelagert. Caprolactamsulfat und überschüssige Schwefelsäure wurden mit Ammoniak neutralisiert.

Das dabei anfallende zweiphasige Gemisch wurde getrennt, und eine wässrige Ammoniumsulfat-Lösung wurde abgetrennt und ausgeschleust.

Die wasserhaltige Rohcaprolactam-Phase wurde mit Toluol wie vorstehend beschrieben extrahiert. Die zweiphasigen Extrakte wurden durch Phasentrennung voneinander getrennt. Die wässrige Phase wurde entsorgt. Aus der organischen Phase, die das Caprolactam enthielt, wurde das Toluol nach Zusatz von 2,5 mmol NaOH/kg Caprolactam destillativ abgetrennt und in die Extraktionsstufe zurückgeführt. Das so erhaltene Extraktlactam enthielt 4 bis 10 Gew.-% Wasser.

### Beispiele 2a bis 2f

In Beispiel 1 erhaltenes Extraktlactam mit einem Wassergehalt von 4 bis 10 Gew.% wurde mit den in Tabelle 1 angegebenen Mengen an 2,5-Gew.-%-iger wässriger Natronlauge (1,25 bis 10 mmol NaOH pro kg Extraktlactam) versetzt. In zwei hintereinander geschalteten Verdampfern wurde das Extraktlactam wie beschrieben entwässert und bei vermindertem Druck in zwei Ko-Ionnen destillativ gereinigt. In der ersten Kolonne wurden Wasser und niedriger als Caprolactam siedende Verunreinigungen als Kopfprodukte entfernt.

Das Sumpfprodukt der ersten Kolonne wurde in einer zweiten Destillationskolonne weiter gereinigt. Dabei wurden gegenüber Caprolactam hochsiedende Verunreinigungen über Sumpf abgezogen, und es wurde spezifikationsgerechtes Caprolactam gewonnen.

**Tabelle 1**

| Beispiele | mmol NaOH / kg Capro. | PAN | E₂₉₀ | VB meqOH- /kg | Alkalinität meqOH⁻ /kg |
|---|---|---|---|---|---|
| 2a | 10 | 1,8 | 0,035 | 0,10 | 0,012 |
| 2b | 7,5 | 1,5 | 0,036 | 0,13 | 0,013 |
| 2c | 5,0 | 1,3 | 0,040 | 0,10 | 0,010 |
| 2d | 2,5 | 1,5 | 0,044 | 0,10 | 0,012 |
| 2e | 1,75 | 1,9 | 0,049 | 0,11 | 0,010 |
| V2f | 1,25 | 1,9 | 0,053 | 0,11 | 0,011 |

Die Bestimmung der Werte für PAN, E₂₉₀, VB (volatile bases) erfolgte wie in EP-B-1 423 361 in Tabelle 1 beschrieben, der Alkalinität durch Titration mit einer 0,01 molaren wässrigen HCl-Lösung gegen Tashiro-Indikator.

In Ullmann's Encyclopedia of Industrial Chemistry, 6. Aufl., Band 6, Seite 200 werden für die Caprolactam-Standard-Spezifikation folgende Werte angegeben:
- Permanganate absorption number (PAN): max 5
- Absorbance 290 nm (E₂₉₀): max 0,05
- Volatile bases (VB): max 0,5 meq/kg

Die Grenzwerte werden erfindungsgemäß eingehalten.

Der Vergleich zwischen Tabelle 1 in EP 1 423 361 B1 und der vorstehenden Tabelle 1 zeigt:
- Trotz der geringeren Zahl an Reinigungsstufen im erfindungsgemäßen Verfahren sind die für eine Einhaltung der Caprolactam-Spezifikation benötigten Natronlauge-Mengen nur unwesentlich höher als in EP 1 423 361 B1 (Beispiel 2e: 1,75 mmol NaOH/kg Caprolactam gegenüber 0,3 mmol NaOH/kg Caprolactam in EP 1 423 361 B1).
- Außerdem liefert das erfindungsgemäße Verfahren bessere PAN- und E₂₉₀-Werte als EP 1 423 361 B1.
- Schließlich sind - im Unterschied zur vorliegenden Erfindung - die E₂₉₀-Werte in EP 1 423 361 B1, Beispiele II bis IX, nicht spezifikationsgerecht.
- Eine Verbesserung der PAN- und E₂₉₀-Werte mit abnehmender NaOH-Menge wird zwar in EP 1 423 361 B1, nicht aber gemäß der vorliegenden Erfindung beobachtet. In Beispiel V2f wird bei 1,25 mmol NaOH/kg Caprolactam die E₂₉₀-Spezfikation nicht erfüllt.

## Patentansprüche

1. Verfahren zur Herstellung von gereinigtem Caprolactam, umfassend die Schritte
a) Extraktion von Roh-Caprolactam, erhalten aus der Beckmann-Umlagerung von Cyclohexanonoxim, mit einem organischen Extraktionsmittel,
b) Abtrennung der organischen Phase aus Schritt a),
c) destillative Abtrennung des organischen Extraktionsmittels von der organischen Phase aus Schritt b) unter Erhalt von wasserhaltigem Extraktlactam, wobei vor der destillativen Abtrennung wässrige Alkalihydroxid-Lösung in einer Menge von 0,5 bis 6 mmol Alkalihydroxid/kg Caprolactam zugesetzt wird,
d) Zusetzen von 0 bis 30 mmol Alkalihydroxid/kg Caprolactam zum wasserhaltigen Extraktlactam aus Schritt c),
e) destillatives Entwässern des mit Alkalihydroxid versetzten wasserhaltigen Extraktlactams aus Schritt d),
f) Befreien des Caprolactams aus Schritt e) von niedriger und höher als Caprolactam siedenden Nebenprodukten durch Destillation,
wobei in Schritten c) und d) zusammen mindestens 1,5 mmol Alkalihydroxid/kg Caprolactam zugesetzt werden.

2. Verfahren nach Anspruch 1, **dadurch gekennzeichnet, dass** in Schritt d) 1,5 bis 30 mmol Alkalihydroxid/kg Caprolactam zugesetzt werden.

3. Verfahren nach Anspruch 1 oder 2, **dadurch gekennzeichnet, dass** in Schritt e) die Verweilzeit weniger als 30 Minuten beträgt.

4. Verfahren nach einem der Ansprüche 1 bis 3, **dadurch gekennzeichnet, dass** das organische Lösungsmittel in Schritt a) Toluol oder Benzol ist.

5. Verfahren nach einem der Ansprüche 1 bis 4, **dadurch gekennzeichnet, dass** in Schritt e) und/oder f) die Sumpftemperatur 160°C nicht überschreitet.

6. Verfahren nach Anspruch 5, **dadurch gekennzeichnet, dass** in Schritt e) der Druck 20 bis 100 mbar (Kopfdruck) in zumindest einer Destillationsstufe beträgt.

7. Verfahren nach Anspruch 5 oder 6, **dadurch gekennzeichnet, dass** in Schritt f) der Druck 2 bis 20 mbar (Kopfdruck) beträgt.

8. Verfahren nach einem der Ansprüche 1 bis 7, **dadurch gekennzeichnet, dass** das in der Beckmann-Umlagerung eingesetzte Cyclohexanonoxim durch Oximierung von Cyclohexanon mit von durch katalytische Hydrierung von Stickstoffmonoxid in Gegenwart von Schwefelsäure erhaltenem Hydroxylammoniumsulfat hergestellt wird.

9. Verfahren nach einem der Ansprüche 1 bis 8, **dadurch gekennzeichnet, dass** in Schritt d) 1,75 bis 14 mmol Alkalihydroxid/kg Caprolactam zugesetzt werden.

10. Verfahren nach einem der Ansprüche 1 bis 9, **dadurch gekennzeichnet, dass** die Gesamtmenge des in Schritten c) und d) zugegebenen Alkalihydroxids maximal 14 mmol/kg Caprolactam beträgt.

## Claims

1. A process for preparing purified caprolactam, comprising the steps
a) extraction of crude caprolactam, obtained from the Beckmann rearrangement of cyclohexanone oxime, with an organic extractant,
b) removal of the organic phase from step a),
c) distillative separation of the organic extractant from the organic phase from step b) giving rise to water-containing lactam extract, the distillative separation being preceded by addition of aqueous alkali metal hydroxide solution in an amount of from 0.5 to 6 mmol of alkali metal hydroxide/kg of caprolactam,
d) addition of 0 to 30 mmol of alkali metal hydroxide/kg of caprolactam to the water-containing lactam extract from step c),
e) distillative removal of water from the water-containing lactam extract treated with alkali metal hydroxide from step d),
f) freeing the caprolactam from step e) from byproducts lower- and higher-boiling than caprolactam by distillation,
with addition in steps c) and d) together of at least 1.5 mmol of alkali metal hydroxide/kg of caprolactam.

2. The process according to claim 1, wherein 1.5 to 30 mmol of alkali metal hydroxide/kg of caprolactam are added in step d).

3. The process according to claim 1 or 2, wherein the residence time is less than 30 minutes in step e).

4. The process according to any of claims 1 to 3, wherein the organic solvent in step a) is toluene or benzene.

5. The process according to any of claims 1 to 4, wherein the bottom temperature in step e) and/or f) does not exceed 160°C.

6. The process according to claim 5, wherein the pressure in step e) is 20 to 100 mbar (top pressure) in at least one distillation stage.

7. The process according to claim 5 or 6, wherein the pressure in step f) is 2 to 20 mbar (top pressure).

8. The process according to any of claims 1 to 7, wherein the cyclohexanone oxime used in the Beckmann rearrangement is prepared by oximation of cyclohexanone with hydroxylammonium sulfate, obtained by catalytic hydrogenation of nitrogen monoxide in the presence of sulfuric acid.

9. The process according to any of claims 1 to 8, wherein 1.75 to 14 mmol of alkali metal hydroxide/kg of caprolactam are added in step d).

10. The process according to any of claims 1 to 9, wherein the total amount of the alkali metal hydroxide added in steps c) and d) is at most 14 mmol/kg of caprolactam.

## Revendications

1. Procédé de préparation de caprolactame purifié, comprenant les étapes consistant à
a) extraire du caprolactame brut, obtenu à partir de la transposition de Beckmann de cyclohexanonoxime, à l'aide d'un agent d'extraction organique,
b) séparer la phase organique de l'étape a),
c) séparer par distillation l'agent d'extraction organique de la phase organique de l'étape b) avec obtention d'un extrait de lactame contenant de l'eau, une solution aqueuse d'hydroxyde de métal alcalin étant ajoutée avant la séparation par distillation en une quantité de 0,5 à 6 mmoles d'hydroxyde de métal alcalin/kg de caprolactame,
d) ajouter 0 à 30 mmoles d'hydroxyde de métal alcalin/kg de caprolactame à l'extrait de lactame contenant de l'eau de l'étape c),
e) déshydrater par distillation l'extrait de lactame contenant de l'eau additionné d'hydroxyde de métal alcalin de l'étape d),
f) libérer par distillation le caprolactame de l'étape e) de produits secondaires présentant un point d'ébullition inférieur et supérieur à celui du caprolactame,
au moins 1,5 mmole d'hydroxyde de métal alcalin/kg de caprolactame étant ajoutée au total dans les étapes c) et d).

2. Procédé selon la revendication 1, **caractérisé en ce qu'**on ajoute dans l'étape d) 1,5 à 30 mmoles d'hydroxyde de métal alcalin/kg de caprolactame.

3. Procédé selon la revendication 1 ou 2, **caractérisé en ce que** le temps de séjour dans l'étape e) est inférieur à 30 minutes.

4. Procédé selon l'une quelconque des revendications 1 à 3, **caractérisé en ce que** le solvant organique dans l'étape a) est le toluène ou le benzène.

5. Procédé selon l'une quelconque des revendications 1 à 4, **caractérisé en ce que** dans l'étape e) et/ou f), la température du fond ne dépasse pas 160°C.

6. Procédé selon la revendication 5, **caractérisé en ce que** dans l'étape e) la pression est de 20 à 100 mbars (pression de tête) dans au moins une étape de distillation.

7. Procédé selon la revendication 5 ou 6, **caractérisé en ce que** dans l'étape f), la pression est de 2 à 20 mbars (pression de tête).

8. Procédé selon l'une quelconque des revendications 1 à 7, **caractérisé en ce que** la cyclohexanonoxime utilisée dans la transposition de Beckmann est préparée par oximation de cyclohexanone avec du sulfate d'hydroxylammonium obtenu par hydrogénation catalytique de monoxyde d'azote en présence d'acide sulfurique.

9. Procédé selon l'une quelconque des revendications 1 à 8, **caractérisé en ce qu'**on ajoute dans l'étape d) 1,75 à 14 mmoles d'hydroxyde de métal alcalin/kg de caprolactame.

10. Procédé selon l'une quelconque des revendications 1 à 9, **caractérisé en ce que** la quantité totale d'hydroxyde de métal alcalin ajouté dans les étapes c) et d) est d'au maximum 14 mmoles/kg de caprolactame.
